Europäisches Patentamt

European Patent Office

Office européen des brevets

(11) Numéro de publication : **0 338 890 B1**

## (12) FASCICULE DE BREVET EUROPEEN

(45) Date de publication du fascicule du brevet :
**09.09.92 Bulletin 92/37**

(51) Int. Cl.$^5$ : **C07C 25/18, C07C 17/12**

(21) Numéro de dépôt : **89400999.2**

(22) Date de dépôt : **12.04.89**

(54) **Procédé de préparation du bromo-4 biphényle.**

(30) Priorité : **22.04.88 FR 8805331**

(43) Date de publication de la demande :
**25.10.89 Bulletin 89/43**

(45) Mention de la délivrance du brevet :
**09.09.92 Bulletin 92/37**

(84) Etats contractants désignés :
**AT BE CH DE ES FR GB GR IT LI LU NL SE**

(56) Documents cités :
**DE-A- 3 318 356**
**CHEMICAL ABSTRACTS, vol. 93, no. 7, 18 août 1980, page 929, abstract no. 71251p, Columbus, Ohio, US; PL-A-105 774**

(56) Documents cités :
**JOURNAL OF ORGANIC CHEMISTRY OF THE USSR, vol. 24, no. 2, partie 1, février 1988, pages 217-223, Plenum Publishing Corp., NewYork, US; A.V. CHEPRAKOV et al.: "Catalytic and stoichiometric brominatin of aromatic compounds in aqueous trifluoroacetic acid inthe presence of nitrogen-containing oxidizing agents"**

(73) Titulaire : **RHONE-POULENC CHIMIE**
**25, quai Paul Doumer**
**F-92408 Courbevoie Cédex (FR)**

(72) Inventeur : **Nonn, Alain**
**11, Allée de la Gravière**
**F-69110 Sainte Foy Les Lyon (FR)**

(74) Mandataire : **Dubruc, Philippe et al**
**RHONE-POULENC CHIMIE Service Brevets Chimie 25, quai Paul-Doumer**
**F-92408 Courbevoie Cédex (FR)**

## Description

La présente invention concerne la préparation du bromo-4 biphényle.

Le bromo-4 biphényle est un produit susceptible de nombreuses applications.

Il peut être utilisé dans la fabrication de composés agrochimiques ou pharmaceutiques. Il peut aussi servir à la préparation de cristaux liquides.

Le problème de sa synthèse est qu'il faut réaliser une monobromation du biphenyle et donc éviter au maximum la formation de dibromo biphenyle et ceci en gardant un taux de transformation important du biphényle. Il était déjà connu de réaliser la bromation sélective en utilisant comme solvant de l'anhydride acétique (CHEMICAL ABSTRACT, vol. 93, N° 7, 18 août 1980, page 929, abstract N° 71251p, Colombus, Ohio, U.S.) ; toutefois, utiliser comme solvant un composé tellement réactif qu'il est quasiment impossible de l'utiliser de manière économique au plan industriel.

L'objet principal de l'invention est donc un procédé qui permet de résoudre ce problème tout en étant industriel.

Dans ce but, le procédé de préparation selon l'invention du bromo-4 biphényle est caractérisé en ce qu'on fait réagir le biphényle avec du brome dans un milieu comprenant un solvant choisi dans le groupe comprenant les composés organo-soufrés, les amides, les nitriles, les acides dont le pKa est d'au moins 3, en ce que la réaction est menée à une température comprise entre 0 et 60°C et en ce que lorsque le milieu comporte en outre comme solvant un acide dont le pKa est inférieur à 3, le rapport entre ledit acide dont le pKa est inférieur à 3 et le mélange total des acides de pKa d'au moins 3 et d'acides de pKa inférieurs à 3 est d'au plus 50%.

Ce procédé est d'autant plus surprenant que selon le titre DE-A-3 318 356 des techniques très similaires à celles de la présente invention conduisaient à une synthèse exclusive du dérivé de bromé.

Le procédé de l'invention permet, à température ambiante, d'obtenir du bromo-4 biphényle avec un rendement supérieur à au moins 65 % et dans certains cas à au moins 75 %.

D'autres caractéristiques et avantages de l'invention seront mieux compris à la lecture de la description qui va suivre et des exemples concrets mais non limitatifs de mise en oeuvre du procédé.

La caractéristique principale de l'invention réside dans le choix du solvant du milieu réactionnel. Celui-ci devra être inerte vis-à-vis du brome.

Le solvant peut tout d'abord appartenir au groupe des composés organo-soufrés.

Parmi ceux-ci on peut citer plus particulièrement le disulfure de carbone, le diméthylsulfoxyde, les sulfolanes.

Un autre groupe de solvants qui peut être mis en oeuvre dans le procédé de l'invention est celui des amides.

On peut mentionner notamment le N, N-diméthylformamide (DMF), le N,N-dimethylacétamide, le N-méthylacétamide, les dérivés de la pipéridine comme la formyl-1 pipéridine, la N-méthylpyrolidone.

Le solvant peut aussi être choisi dans le groupe des nitriles. On peut citer parmi ceux-ci l'acétonitrile, le n-butyronitrile, le lactonitrile.

Un dernier groupe est celui constitué par les acides dont le pKa est supérieur à 3. Selon un mode de réalisation particulier de l'invention on utilise les acides dont le pKa est d'au moins 4.

Les acides sont de préférence les acides carboxyliques.

On peut utiliser ainsi l'acide acétique, l'acide propionique.

Bien entendu, on peut envisager d'opérer avec un milieu réactionnel comprenant un mélange des solvants précités.

Il est à noter que le procédé de l'invention peut être mis en oeuvre dans un milieu à base d'un mélange d'eau et d'au moins un des solvants cités ci-dessus. Dans un tel cas on peut observer une amélioration nette de la cinétique de réaction.

Selon un mode de réalisation particulier de l'invention le milieu réactionnel comprend au moins un acide notamment carboxylique du type décrit ci-dessus mais en outre la réaction est conduite en présence d'un système catalytique à base d'un composant choisi dans le groupe comprenant les acides de Lewis et l'iode.

Comme acide de Lewis on utilise plus particulièrement ceux comprenant un élément appartenant aux groupes IIb, IIIa, IVb, Va, Vb, VIa et VIII de la classification périodique. On peut mentionner dans ce cas le fer, le zinc, l'antimoine, le tellurium, le zirconium, le titane, l'aluminium, le gallium, l'hafnium, le nobium, le phosphore, le bore.

Plus précisément, on met en oeuvre les halogénures de ces éléments tels que par exemple $FeCl_3$, $ZnCl_2$, $SbCl_5$, $PBr_3$, $AlCl_3$, $TiCl_4$, $Z_rCl_4$.

En général le système catalytique utilisé comprend un seul composant du type qui vient d'être décrit. Toutefois, il est possible d'utiliser un système à base d'au moins deux de ces composants.

D'une manière générale, la quantité de catalyseur sera comprise entre 0,1 et 10 % exprimée en mole par rapport au biphényle engagé et plus particulièrement entre 0,5 et 2 %.

Enfin, selon un autre mode de réalisation de l'invention, il est possible de réaliser la réaction de bromation dans un milieu comprenant, outre un acide de pKa d'au moins 3 du type étudié ci-dessus, un acide de pKa inférieur à 3.

Pour ce dernier type d'acide, on peut mentionner notamment ceux choisis dans le groupe comprenant les acides carboxyliques ou sulfoniques. Plus particulièrement, on emploiera les acides substitués par un ou plusieur halogènes, notamment ceux substitués par le fluor.

A titre d'exemple, on citera l'acide trifluoroacétique, l'acide trifluorométhane sulfonique.

Dans ce mode de réalisation, il est à noter que la proportion en volume d'acide de pKa inférieur à 3 est d'au plus 50 % du mélange total acide de pKa d'au moins 3 - acide de pKa inférieur à 3.

La réaction se fait généralement en coulant le brome dans un mélange comprenant le biphényle, le solvant et le système catalytique.

On utilise habituellement une excès de brome compris entre 0 et 120 % par rapport à la stoechiométrie plus particulièrement d'au moins 50 %, ceci notamment dans le cas d'un système catalytique à au moins deux composants précités.

On a pu vérifier que malgré un excès de brome et même avec des temps de réaction longs (ceux-ci peuvent varier entre environ 10 et 30 heures) il n'y avait pas formation importante de dérivé dibromé.

La réaction est conduite à une température qui peut varier entre 0 et 60°C. On préfère travailler à température ambiante.

Le produit bromé formé peut enfin être séparé par tout moyen connu, notamment par cristallisation.

Des exemples de mise en oeuvre du procédé de l'invention vont être donnés ci-dessous.

EXEMPLE 1

Dans un réacteur de 250 ml muni d'une agitation magnétique, d'un réfrigérant, d'une ampoule de coulée, d'un thermomètre, on charge
  – 15,4 g de biphényle (0,1 M),
  – 100 ml de solvant
à température ambiante, on coule en 10 minutes le brome (39,2 g - 0,22 M) puis on agite un temps variable à température ambiante. En fin de réaction, l'excès de brome est détruit par du sulfite ou du bisulfite de sodium ou de potassium. La phase organique est lavée, séchée et diluée à un volume donné pour être analysée en CPG.

Les résultats sont donnés dans le tableau 1 (page 5).

EXEMPLE 2

On procède comme dans l'exemple 1 mais avec l'acide acétique comme solvant et avec un système catalytique. Les résultats sont donnés dans le tableau 2 (page 6).

EXEMPLE 3

Dans le même appareillage que celui de l'exemple 1, on charge :
  – 7,72 g (0,05 mole) de biphényle
  – 45 ml d'acide acétique
  – 5 ml d'acide trifluoroacétique
On coule en 18 minutes à température ambiante 17,6 g (0,11 mole) de brome.
On procède ensuite comme dans les exemples précédents.
Les résultats sont donnés ci-dessous :

```
                                        Rendement
Temps de réaction                          8 h
. Bromo-2 biphényle                       3,0 %
. Bromo-4 biphényle                      78,1 %
. Dibromo-4,4'biphényle                  17,0 %
```

TABLEAU 1

| ESSAI | SOLVANT | DUREE (H) | RENDEMENT % | | | |
|---|---|---|---|---|---|---|
| | | | Br-2 | Br-4 | di Br 2,4' | di Br 4,4' |
| 1 | AcOH | 29 | 4,4 | 74,0 | 1,0 | 17,0 |
| 2 | AcOH 30 $H_2O$ 70 | 8 | 3,1 | 68,8 | 1,2 | 29,1 |
| 3 | $CS_2$ | 21 | 5,9 | 86,5 | 0 | 4,9 |
| 4 | DMF | 21 | 7,8 | 77,9 | 1,3 | 15,9 |
| 5 | $CH_3CN$ | 24 | 3,1 | 66,0 | 1,1 | 27,8 |

EP 0 338 890 B1

TABLEAU 2

| ESSAI | SOLVANT | CATALYSEUR | DUREEE | RENDEMENT % | | | |
|-------|---------|------------|--------|------|------|----------|-----------|
| | | | | Br-2 | Br-4 | di Br-2,4 | di Br-4,4' |
| 6 | AcOH | $I_2$(1 %) | 28 h | 4,4 | 75,5 | 1,0 | 14,4 |
| 7 | AcOH | $ZnCl_2$(1 %) | 25 h | 5,1 | 85,5 | 1,1 | 15,8 |
| 8 | AcOH | $FeCl_3$(1 %) | 7 h | 4,4 | 79,5 | 0 | 13,7 |

EP 0 338 890 B1

Bien entendu, l'invention n'est nullement limitée aux modes de réalisation décrits qui n'ont été donnés qu'à titre d'exemples. En particulier, elle comprend tous les moyens constituant des équivalents techniques des moyens décrits ainsi que leurs combinaisons si celles-ci sont mises en oeuvre dans le cadre de la protection comme revendiquée.

**Revendications**

1 - Procédé de préparation du bromo-4 biphényle caractérisé en ce qu'on fait réagir le biphényle avec du brome dans un milieu comprenant un solvant choisi dans le groupe comprenant les composés organo-soufrés, les amides, les nitriles, les acides dont le pKa est d'au moins 3, en ce que la réaction est menée à une température comprise entre 0 et 60°C et en ce que lorsque le milieu comporte en outre comme solvant un acide dont le pKa est inférieur à 3, le rapport entre ledit acide dont le pKa est inférieur à 3 et le mélange total des acides de pKa d'au moins 3 et d'acides de pKa inférieurs à 3 est d'au plus 50%.

2 - Procédé selon la revendication 1, caractérisé en ce que le milieu comprend de l'eau en plus des solvants précités.

3 - Procédé selon l'une des revendications 1 ou 2, caractérisé en ce que les acides présentent un pKa d'au moins 4.

4 - Procédé selon l'une des revendication précédentes, caractérisé en ce que les acides sont les acides carboxyliques.

5 - Procédé selon l'une des revendications précédentes, caractérisé en ce que le solvant comprend au moins un acide et en ce que la réaction est conduite en présence d'un système catalytique à base d'au moins un composant choisi dans le groupe comprenant les acides de Lewis et l'iode.

6 - Procédé selon la revendication 5, caractérisé en ce qu'on choisit les acides de Lewis parmi ceux comprenant un élément appartenant aux groupes IIb, IIIa, IVb, Va, Vb, VIa et VIII de la classification périodique.

7 - Procédé selon les revendications 5 ou 6, caractérisé en ce qu'on utilise comme acide de Lewis les halogénures des éléments précités.

8 - Procédé selon l'une des revendications 5 à 7 caractérisé en ce qu'on utilise un excès de brome d'au moins 50 % par rapport à la stoechiométrie.

9 - Procédé selon l'une des revendications précèdentes caractérisé en ce que le milieu comprend, outre un acide de pKa d'au moins 3, un acide de pKa inférieur à 3.

10 - Procédé selon l'une des revendications 1 à 9, caractérisé en ce que la réaction est menée à température ambiante.

**Patentansprüche**

1. Verfahren zur Herstellung von 4-Brombiphenyl, dadurch gekennzeichnet, daß man das Biphenyl mit Brom in einem Medium umsetzt, das ein Lösungsmittel, ausgewählt aus der Gruppe umfassend die Schwefel-organischen Verbindungen, Amide und Nitrile, die Säuren mit einem pKa von mindestens 3 umfaßt, daß man die Reaktion bei einer Temperatur im Bereich von 0 bis 60°C durchführt und daß, wenn das Medium zusätzlich als Lösungsmittel eine Säure mit einem pKa unterhalb 3 enthält, das Verhältnis zwischen dieser Säure, deren pKa unterhalb 3 liegt, und dem Gesamtgemisch der Säuren mit pKa von mindestens 3 und der Säuren mit pKa unterhalb 3 höchstens 50% ausmacht.

2. Verfahren nach Anspruch 1, dadurch gekennzeichnet, daß das Medium zusätzlich zu den genannten Lösungsmitteln Wasser enthält.

3. Verfahren nach einem der Ansprüche 1 oder 2, dadurch gekennzeichnet, daß die Säuren einen pKa von mindestens 4 aufweisen.

4. Verfahren nach einem der vorangehenden Ansprüche, dadurch gekennzeichnet, daß die Säuren Carbonsäuren sind.

5. Verfahren nach einem der vorangehenden Ansprüche, dadurch gekennzeichnet, daß das Lösungsmittel mindestens eine Säure umfaßt und daß die Reaktion in Gegenwart eines Katalysatorsystems auf der Basis mindestens einer Verbindung, ausgewählt aus der Gruppe umfassend die Lewis Säuren und Iod, durchgeführt wird.

6. Verfahren nach Anspruch 5, dadurch gekennzeichnet, daß man die Lewis Säuren unter denjenigen auswählt, die ein Element aus den Gruppen IIb, IIIa, IVb, Va, Vb, VIa und VIII des Periodensystems enthalten.

7. Verfahren nach den Ansprüchen 5 oder 6, dadurch gekennzeichnet, daß man als Lewis Säure die Halogenide der vorgenannten Elemente einsetzt.

8. Verfahren nach einem der Ansprüche 5 bis 7, dadurch gekennzeichnet, daß man einen Bromüberschuß von mindestens 50%, bezogen auf das stöchiometrische Verhältnis, einsetzt.

9. Verfahren nach einem der vorangehenden Ansprüche, dadurch gekennzeichnet, daß das Medium zusätzlich zu einer Säure mit einem pKa von mindestens 3 eine Säure mit einem pKa unterhalb 3 enthält.

10. Verfahren nach einem der Ansprüche 1 bis 9, dadurch gekennzeichnet, daß man die Reaktion bei Raumtemperatur durchführt.

## Claims

1. Process for the preparation of 4-bromodiphenyl, characterized in that the diphenyl is reacted with the bromine in a medium incorporating a solvent chosen from the group including organosulphurous compounds, amides, nitriles and acids with a pKa of at least 3, in that the reaction is performed at a temperature between 0 and 60°C and that when the medium also contains as the solvent an acid with a pKa below 3, the ratio between the acid with the pKa below 3 and the total mixture of acids with a pKa of at least 3 and acids with a pKa below 3 is at the most 50%.

2. Process according to claim 1, characterized in that the medium includes water, apart from the aforementioned solvents.

3. Process according to either of the claims 1 and 2, characterized in that the acids have a pKa of at least 4.

4. Process according to any one of the preceding claim, characterized in that the acids are carboxylic acids.

5. Process according to any one of the preceding claim, characterized in that the solvent comprises at least one acid and in that the reaction is performed in the presence of a catalytic system based on at least one component chosen in the group including Lewis acids and iodine.

6. Process according to claim 5, characterized in that the Lewis acids are chosen from among those including an element belonging to groups IIb, IIIa, IVb, Va, Vb, VIa and VIII of the periodic system.

7. Process according to claim 5 or 6, characterized in that the Lewis acid used consists of halides of the aforementioned elements.

8. Process according to one of the claims 5 to 7, characterized in that an at least 50% bromine excess with respect to the stoichiometry is used.

9. Process according to any one of the preceding claim, characterized in that the medium comprises, apart from an acid with a pKa of at least 3, an acid with a pKa below 3.

10. Process according to any one of the claims 1 to 9, characterized in that the reaction is performed at ambient temperature.